(19) Europäisches Patentamt / European Patent Office / Office européen des brevets

(11) **EP 3 826 725 B1**

(12) **FASCICULE DE BREVET EUROPEEN**

(45) Date de publication et mention de la délivrance du brevet:
**03.08.2022 Bulletin 2022/31**

(21) Numéro de dépôt: **19744771.7**

(22) Date de dépôt: **25.06.2019**

(51) Classification Internationale des Brevets (IPC):
**A61Q 19/08** *(2006.01)*   **A61K 8/9755** *(2017.01)*
**A61K 8/9767** *(2017.01)*

(52) Classification Coopérative des Brevets (CPC):
**A61K 8/9767; A61K 8/9755; A61Q 19/08**

(86) Numéro de dépôt international:
**PCT/FR2019/051557**

(87) Numéro de publication internationale:
**WO 2020/021175 (30.01.2020 Gazette 2020/05)**

(54) **UTILISATION D'UNE HUILE ESSENTIELLE DE RAMEAUX DE SAPIN BLANC POUR LUTTER CONTRE LES SIGNES DU VIEILLISSEMENT CUTANÉ**

VERWENDUNG EINES ETHERISCHEN ÖLS VON WEISSEN FIR-BAUMZWEIGEN ZUR BEKÄMPFUNG DER ANZEICHEN DER HAUTALTERUNG

USE OF AN ESSENTIAL OIL OF WHITE FIR TREE BRANCHES TO COMBAT THE SIGNS OF SKIN AGEING

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.07.2018 FR 1856839**

(43) Date de publication de la demande:
**02.06.2021 Bulletin 2021/22**

(73) Titulaires:
• **LABORATOIRES M & L**
  **04100 Manosque (FR)**
• **Centre national de la recherche scientifique**
  **75016 Paris (FR)**
• **Université Côte d'Azur**
  **06100 Nice (FR)**

(72) Inventeurs:
• **BOUVILLE, Anne-Sophie**
  **06250 MOUGINS (FR)**
• **FERNANDEZ, Xavier**
  **06100 NICE (FR)**
• **PORTES, Pascal**
  **13540 PUYRICARD (FR)**
• **LHOMMET, Jean-Charles**
  **13013 MARSEILLE (FR)**

(74) Mandataire: **Renard, Emmanuelle**
  **Renard IP**
  **4 Bis Avenue de Lorraine**
  **92380 Garches (FR)**

(56) Documents cités:
**WO-A1-2004/108107     WO-A1-2017/044050**
**JP-A- 2010 132 629     US-A1- 2009 317 342**

• **EVA TAVCAR BENKOVIC ET AL: "Chemical composition of the silver fir (Abies alba) bark extract Abigenol and its antioxidant activity", INDUSTRIAL CROPS AND PRODUCTS., vol. 52, janvier 2014 (2014-01), pages 23-28, XP055577838, NL ISSN: 0926-6690, DOI: 10.1016/j.indcrop.2013.10.005**
• **Gorazd Dreven?ek ET AL: "Silver fir (Abies alba) trunk extract protects guinea pig arteries from impaired functional responses and morphology due to an atherogenic diet", Phytomedicine, vol. 22, no. 9, 1 August 2015 (2015-08-01), pages 856-861, XP055577853, AMSTERDAM, NL ISSN: 0944-7113, DOI: 10.1016/j.phymed.2015.06.004**

**Description**

OBJET DE L'INVENTION

La présente invention concerne l'utilisation d'une huile essentielle de rameaux de sapin blanc *(Abies alba)* comme inhibiteur d'élastase pour lutter contre les signes du vieillissement cutané, en particulier pour limiter et/ou réduire la dégradation des fibres élastiques de la peau, le relâchement cutané et/ou la perte d'élasticité de la peau et/ou l'apparence des rides.

ARRIERE-PLAN DE L'INVENTION

La peau comprend notamment deux compartiments, l'un superficiel, l'épiderme, et l'autre plus profond, le derme, qui interagissent. L'épiderme humain naturel est composé principalement de trois types de cellules que sont les kératinocytes, très majoritaires, les mélanocytes, et les cellules de Langerhans. Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale, comprenant des glycosaminoglycanes sulfatés (ex. chondoitine sulfate) ou pas (ex. acide hyaluronique), des protéoglycanes et diverses protéases. Ces composants sont synthétisés par les fibroblastes. On y trouve aussi des leucocytes, des mastocytes ou encore des macrophages tissulaires. Enfin, le derme est traversé par des vaisseaux sanguins et des fibres nerveuses. La cohésion entre l'épiderme et le derme est assurée par la jonction dermo-épidermique.
Parmi les constituants du derme, l'élastine est une protéine qui joue un rôle essentiel dans l'élasticité et la souplesse de la peau.
On sait que lors d'un stress cutané superficiel, qui peut notamment être d'origine chimique, physique ou bactérienne, les kératinocytes des couches superficielles de l'épiderme libèrent des médiateurs biologiques qui possèdent la capacité d'attirer certaines cellules infiltrantes de la peau, elles-mêmes responsables de l'entretien d'une irritation locale transitoire. Ces cellules infiltrant les zones irritées ou agressées libèrent alors des enzymes parmi lesquelles on peut citer l'élastase leucocytaire. Sous l'action de cette enzyme, les fibres élastiques de soutien extracellulaire du tissu conjonctif peuvent être dégradées. Ainsi, à long terme, la somme des micro-stress cutanés superficiels, par exemple générés par une exposition prolongée aux UV ou par des agents irritants, peut entraîner une déstructuration du réseau formé par les fibres élastiques du tissu conjonctif. Il s'ensuit une perte d'élasticité et de souplesse de la peau, ainsi qu'une accentuation des rides.
La présente invention a pour but de proposer une solution à ce problème, et notamment de proposer de nouveaux composés susceptibles d'être utilisés en cosmétique pour limiter le vieillissement de la peau, et notamment le vieillissement généré par la dégradation de l'élastine.
Dans cet objectif, les Demandeurs ont mis en évidence qu'une huile essentielle de sapin blanc *(Abies alba)* présentait une activité anti-élastase supérieure à celle d'autres extraits végétaux censés agir sur cette cible biologique.
Le sapin blanc, encore désigné par sapin commun ou sapin pectiné, est connu sous le terme botanique d'*Abies alba* Mill. (ou *Abies pectinata* Lam. DC ou *Abies excelsa* Lam. ou *Pinus picea* L. ou *Abies picea* L.). Il s'agit d'un conifère originaire d'Europe sur les rameaux duquel se dressent des aiguilles (ou feuilles) sempervirentes, ainsi que des cônes (pommes de pin) pourvus de bractées.
L'huile essentielle de cônes de sapin blanc est connue en parfumerie (D.L.J. Opdyke, Monographs on Fragrance Raw Materials, Pergamon Press, 1979). Par ailleurs, l'huile essentielle obtenue par entraînement à la vapeur des branches et aiguilles de sapin blanc originaire d'Asie a été décrite comme présentant des propriétés anti-radicalaires (SA Yang et al., Journal of Clinical Biochemistry and Nutrition, 2009, 44(3), 253-259). Il n'a toutefois pas été suggéré d'effet anti-élastase de cette huile essentielle permettant d'envisager son utilisation dans le traitement du vieillissement cutané.
Par ailleurs, il a été suggéré d'utiliser des extraits de branches et/ou feuilles de sapin blanc, autres que l'huile essentielle, à des fins cosmétiques. En particulier, la demande WO 2017/044050 divulgue l'utilisation comme actifs antirides de polyphénols obtenus par extraction à l'eau bouillante des branches de sapin blanc et récupération de la phase aqueuse. De son côté, le document FR 1 525 945 divulgue l'utilisation comme actif anti-âge d'un extrait de feuilles de sapin blanc obtenu par extraction alcoolique.
Enfin, les propriétés anti-élastase d'extraits d'un sapin autre que le sapin blanc ont été décrites dans la demande de brevet JP 2014-224073. Le procédé décrit dans ce document utilise en particulier un extrait glycolique de pin Todo (*Abies mariesii*).
Ainsi, à la connaissance des Demandeurs, il n'a encore jamais été suggéré que l'huile essentielle de rameaux de sapin blanc était capable d'inhiber l'élastase et pouvait de ce fait être utilisée dans une composition cosmétique destinée à lutter contre les signes du vieillissement cutané.

RESUME DE L'INVENTION

L'invention a pour objet l'utilisation d'une huile essentielle de rameaux de sapin blanc *(Abies alba)* comme inhibiteur d'élastase pour lutter contre les signes du vieillissement cutané.

L'invention a également pour objet un procédé cosmétique pour lutter contre les signes du vieillissement cutané, notamment pour limiter et/ou réduire la dégradation des fibres élastiques de la peau, le relâchement cutané et/ou la perte d'élasticité de la peau et/ou l'apparence des rides, comprenant l'application topique sur la peau d'une composition cosmétique renfermant une huile essentielle de rameaux de sapin blanc *(Abies alba).*

DESCRIPTION DETAILLEE

La présente invention met en œuvre une huile essentielle de rameaux de sapin blanc.

Par "rameaux" on entend dans la présente description l'ensemble des branches et des aiguilles qu'elles portent, à l'exclusion des cônes. Par "huile essentielle", on entend dans cette description le produit de distillation, à savoir de distillation sèche ou d'hydrodistillation, ou d'entraînement à la vapeur des composés organiques volatils présents dans les rameaux du sapin blanc. Les procédés d'hydrodistillation et d'entraînement à la vapeur sont préférés pour une utilisation dans la présente invention. Dans ces procédés, les molécules odorantes contenues dans la plante sont libérées et entraînées mécaniquement avec la vapeur d'eau qui est soit formée par ébullition de l'eau à laquelle la plante est ajoutée (hydrodistillation classique), soit formée par ébullition de l'eau contenue dans la plante (hydrodistillation par micro-ondes), soit encore fournie par une chaudière et mise en circulation à travers la plante (entraînement à la vapeur d'eau). Ces procédés peuvent éventuellement être conduits sous vide ou sous pression.

Plus préférentiellement, l'huile essentielle utilisée selon l'invention est obtenue par hydrodistillation. Le procédé d'hydrodistillation peut être conduit pendant une durée de 1 à 48h, de préférence de 3 à 24h et, mieux, de 6 à 12h. Il a en effet été observé que l'activité anti-élastasique de l'huile essentielle de sapin blanc diminuait lorsque la durée de la distillation était supérieure à 2 jours.

Cette huile essentielle est avantageusement issue de rameaux de sapin blanc cultivé en Europe, de préférence en France et se différencie ainsi par sa composition de l'huile essentielle d'autres espèces de sapin, ou de sapin de la même espèce mais originaire d'Asie, par la présence majoritaire de limonène, d'alpha-pinène et de camphène (représentant au total plus de 50% de l'huile essentielle) et sa teneur modeste en acétate de bornyle.

Plus précisément, l'huile essentielle utilisée selon l'invention renferme, comme constituant principal, du limonène, et, comme constituants secondaires, de l'alpha-pinène, du camphène et de l'acétate de bornyle, dans un rapport molaire du limonène à l'acétate de bornyle allant de 2:1 à 20:1, de préférence de 5:1 à 10:1, tel que mesuré par chromatographie en phase gazeuse.

De son côté, le rapport molaire du camphène à l'alpha-pinène est généralement compris entre 0,5:1 et 2:1, de préférence entre 1:1 et 1,5:1 et/ou le rapport molaire du camphène à l'acétate de bornyle est compris entre 1:1 et 5:1, de préférence entre 2:1 et 3:1, tels que mesurés par chromatographie en phase gazeuse.

L'huile essentielle de rameaux de sapin blanc permet de lutter contre les signes du vieillissement cutané en inhibant l'élastase leucocytaire et peut plus particulièrement être utilisée pour limiter et/ou réduire la dégradation des fibres élastiques de la peau, le relâchement cutané et/ou la perte d'élasticité de la peau et/ou l'apparence des rides.

Elle est généralement incluse dans une composition cosmétique adaptée à une application topique sur la peau. L'huile essentielle de rameaux de sapin blanc représente avantageusement de 0,001 à 5% en poids et préférentiellement de 0,01 à 0,5% en poids, par rapport au poids total de la composition selon l'invention.

Cette composition renferme généralement un milieu physiologiquement acceptable, en particulier cosmétiquement acceptable, c'est-à-dire qui ne génère pas de picotements ou de rougeurs incompatibles avec une utilisation cosmétique, qui ne présente pas d'odeur désagréable et qui est compatible avec la voie d'administration topique. Ce milieu renferme de préférence une phase grasse éventuellement associée à une phase aqueuse. La composition peut ainsi se présenter sous forme anhydre (renfermant au plus 5% en poids d'eau), sous forme d'émulsion huile-dans-eau ou eau-dans-huile ou d'une dispersion d'huile dans l'eau.

Lorsqu'elle est présente, la phase aqueuse renferme de l'eau et éventuellement au moins un constituant choisi parmi les polyols et les gélifiants aqueux. L'eau représente avantageusement de 40 à 80% du poids total de la composition. Le polyol peut notamment être choisi parmi la glycérine, le propylène glycol, le butylène glycol, le pentylène glycol et leurs mélanges et il peut représenter de 3 à 30% du poids total de la composition.

Par "gélifiant aqueux", on désigne un composé polymérique capable d'immobiliser des molécules d'eau en s'hydratant et d'augmenter ainsi la viscosité de la phase aqueuse. Un tel gélifiant peut être choisi parmi : les polysaccharides, tels que : la cellulose et ses dérivés, les amidons modifiés, le carraghénane, l'agar agar, la gomme de xanthane et les gommes végétales telles que la gomme de guar ou de caroube ; les polymères synthétiques et notamment les homopolymères d'acrylate de sodium éventuellement réticulés, ainsi que les copolymères acryliques, en particulier les copolymères d'acrylate de sodium et/ou de (méth)acrylate d'alkyle et/ou de (méth)acrylate d'hydroxyalkyle et/ou de (méth)acrylate de (polyéthoxy)alkyle, avec éventuellement au moins un autre monomère, avantageusement l'acide 2-acrylamido-2-méthylpropane sulfonique (AMPS), ces copolymères étant éventuellement réticulés; et leurs mélanges.

De son côté, la phase grasse peut comprendre une ou plusieurs huiles volatiles et/ou non volatiles. Des exemples d'huiles volatiles sont les alcanes ramifiés, tels que l'isododécane, et les alcanes linéaires en $C_{10}$-$C_{13}$. Comme huiles non volatiles, on peut citer notamment :

- les esters d'acides et de mono-alcool choisis parmi : les mono- et polyesters d'acides linéaires saturés en C2-C10 (de préférence en C6-C10) et de mono-alcools linéaires saturés en C10-C18 (de préférence C10-C14), les mono- et polyesters d'acides linéaires saturés en C10-C20 et de mono-alcools ramifiés ou insaturés en C3-C20 (de préférence C3-C10) ; les mono- et polyesters d'acides ramifiés ou insaturés en C5-C20 et de mono-alcools ramifiés ou insaturés en C5-C20 ; les mono- et polyesters d'acides ramifiés ou insaturés en C5-C20 et de mono-alcools linéaires en C2-C4 ;
- les triglycérides d'acides gras en C6-C12, tels que les triglycérides d'acides caprylique et caprique et la triheptanoïne ;
- les acides gras ramifiés et/ou insaturés en C10-C20 (tels que les acides linoléique, laurique et myristique) ;
- les alcools gras ramifiés et/ou insaturés en C10-C20 (tels que l'octyldodécanol et l'alcool oléylique) ;
- les hydrocarbures tels que le squalane (C30), notamment le squalane végétal extrait de l'huile d'olive, et l'hémisqualane (C15) ;
- les carbonates de dialkyle, tels que le dicaprylyl carbonate et le diéthylhexyl carbonate ;
- les dialkyléthers tels que le dicaprylyl éther ; et
- leurs mélanges.

On peut également citer les huiles végétales qui contiennent un ou plusieurs des constituants précités.

Comme esters d'acides et de monoalcools, on peut notamment citer les monoesters tels que le mélange de caprate et caprylate de coco, le macadamiate d'éthyle, l'ester éthylique de beurre de karité, l'isostéarate d'isostéaryle, l'isononanoate d'isononyle, l'isononanoate d'éthylhexyle, le néopentanoate d'hexyle, le néopentanoate d'éthylhexyle, le néopentanoate d'isostéaryle, le néopentanoate d'isodécyle, le myristate d'isopropyle, le myristate d'octyldodécyle, le palmitate d'isopropyle, le palmitate d'éthylhexyle, le laurate d'hexyle, le laurate d'isoamyle, le nonanoate de cétostéaryle, le capylate de propylheptyle et leurs mélanges. D'autres esters utilisables sont les diesters d'acides et de monoalcools tels que l'adipate de disopropyle, l'adipate de diéthylhexyle, le sébaçate de diisopropyle et le sébaçate de diisoamyle.

Des exemples d'huiles végétales sont notamment les huiles de germe de blé, de tournesol, d'argan, d'hibiscus, de coriandre, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de cassis, d'onagre, de lavande, de bourrache, de millet, d'orge, de quinoa, de seigle, de carthame, de bancoulier, de passiflore, de rosier muscat, d'Echium, de cameline ou de camélia.

La phase grasse peut en outre comprendre au moins un structurant de phase grasse. Par "structurant de phase grasse", on entend un composé capable d'épaissir les huiles contenues dans la composition, choisi notamment parmi les cires, les gélifiants de phase grasse et les corps gras pâteux, ainsi que leurs mélanges.

Selon une forme d'exécution préférée, cette composition renferme en outre au moins un actif anti-âge, en particulier un actif adapté à prévenir et/ou traiter les rides, le relâchement cutané et/ou la formation de taches pigmentaires, qui peut notamment être choisi parmi les agents anti-radicalaires, les agents stimulant la différenciation et/ou la prolifération des kératinocytes et/ou des fibroblastes ; les agents stimulant la synthèse de glycosaminoglycanes et/ou de collagène et/ou de fibrilles d'ancrage dermo-épidermique ; les agents prévenant la dégradation du collagène et/ou des glycosaminoglycanes et/ou des fibrilles d'ancrage dermo-épidermique ; les agents anti-glycation ; les agents dépigmentants et/ou inhibant la mélanogénèse ; et leurs mélanges.

Des exemples de tels actifs anti-âge sont notamment : l'acide ascorbique, ses sels, ses éthers et ses esters, notamment le glucoside d'ascorbyle ; l'adénosine ; le ribose ; les extraits de miel ; les protéines et glycoprotéines, extraites notamment d'amande douce ; les protéines végétales hydrolysées, notamment issues du riz, des graines d'hibiscus ou du lupin ; les polypeptides et les pseudodipeptides, tels que le chlorhydrate de carcinine, le palmitoyl pentapeptide-4 (Pal-Lys-Thr-Thr-Lys-Ser) et le palmitoyl tripeptide-38 commercialisés notamment par SEDERMA sous les dénominations commerciale Matrixyl® 3000 et Matrixyl® Synthe'6, respectivement, le palmitoyl tripeptide-8 commercialisé par la société LUCAS MEYER sous la référence commerciale Nutrazen®, le pentapeptide-18 commercialisé par la société LIPOTEC sous la dénomination commerciale Leuphasyl® Solution, le sh-decapeptide-9 commercialisé par la société SANDREAM sous la dénomination commerciale Neoendorphin® et le palmitoyl hexapeptide-52 commercialisé par la société INFINITEC sous la référence commerciale X50 Myocept® Powder ; les silanes tels que le mannuronate de méthylsilanol ; les arabinoxylanes, extraits en particulier de farine de seigle et les galactoarabinanes, issus notamment du mélèze ; l'acide hyaluronique et ses sels ; les polyphénols, extraits en particulier de mimosa ; les alpha-hydroxyacides, dont ceux extraits de citron; les extraits (généralement aqueux) de plantes telles que le trèfle d'eau, la pensée sauvage, la prêle des champs, la Mafane (*Acmella oleracea*)*,* le chardon aux ânes (*Onopordum acanthium*)*,* le millefeuille (*Achillea*

*millefolium,* contenu notamment dans le produit Neurobiox® de la société BASF), l'embelia (*Embelia concinna,* telle que commercialisée par la société SEPPIC), le figuier de Barbarie (*Opuntia ficus indica,* commercialisé notamment par MIBELLE AG BIOCHEMISTRY sous la dénomination commerciale AquaCacteen), la sauge (*Salvia officinalis,* vendue notamment par PROVITAL GROUP), *Vitex negundo* (commercialisé notamment par les LABORATOIRES EXPANSCIENCE sous la référence commerciale Neurovity®), la châtaigne, la papaye, l'arganier, l'avoine, le tournesol, la pâquerette, la pivoine ou l'aneth ; les extraits aqueux d'algues et notamment de coralline, de janie rouge, *d'Ungaria pinnatifada, d'Alaria esculenta* ou de *Nannochlorosis oculata* ; les huiles essentielles, notamment de myrte ; les gluconates de zinc et/ou de cuivre ; et leurs mélanges.

En variante ou en plus, la composition utilisée selon l'invention peut comprendre au moins un polymère tenseur, c'est-à-dire capable de tendre la peau par action mécanique et de réduire ainsi l'apparence des rides et des ridules. Il peut s'agir d'un polymère synthétique ou naturel, en particulier d'un polysaccharide, notamment d'un extrait d'algue ou de plancton marin ou d'une gomme végétale.

La composition selon l'invention peut en outre contenir différents constituants qui peuvent être dispersés dans la phase grasse et/ou dans la phase aqueuse de l'émulsion, pour autant que ceux-ci soient compatibles avec une application topique sur la peau.

Elle peut ainsi renfermer au moins un émulsionnant huile-dans-eau ou eau-dans-huile, généralement non ionique, tel que des esters de polyoxyéthylène, des esters de sorbitane éventuellement polyéthoxylés, des esters d'acides gras et de glycérol éventuellement polyéthoxylés, des éthers d'alcools gras et de sucre tels que les alkyl glucosides, et leurs mélanges. Les émulsionnants peuvent représenter de 2 à 10% du poids total de la composition. En variante, la composition selon l'invention peut être dépourvue d'émulsionnant.

La composition selon l'invention peut également comprendre une ou plusieurs charges pulvérulentes, qui se présentent avantageusement sous forme de microparticules poreuses ou creuses, de préférence poreuses. Ces microparticules sont en principe sensiblement sphériques. Ces charges peuvent notamment être choisies parmi :

-    les charges organiques telles que : les poudres de polysaccharides et en particulier d'amidon natif, d'amidon modifié ou de cellulose ; les poudres de polymères acryliques tels que le poly(méthacrylate de méthyle), de polyamides ou de polyoléfines ; les poudres d'algues séchées telles que *Corallina officinalis ;*
-    les charges inorganiques telles que la silice, les argiles, la perlite et le talc ;
-    et leurs mélanges.

Ces charges peuvent représenter de 1 à 5% en poids, par rapport au poids total de la composition.

La composition selon l'invention peut en outre comprendre des additifs choisis notamment parmi des agents photoprotecteurs organiques et/ou inorganiques, actifs dans l'UVA et/ou l'UVB, des parfums, des agents anti-oxydants, des agents séquestrants, des ajusteurs de pH, des conservateurs, des pigments, des colorants, et leurs mélanges.

Des exemples non limitatifs de filtres UV comprennent : les benzophénones, telles que celle identifiée par le nom INCI DIETHYLAMINO HYDROXYBENZOYL HEXYL BENZOATE (Uvinul® A Plus de BASF) et la BENZOPHENONE-3 ; les benzotriazoles, tels que celui identifié par le nom INCI METHYLENE BIS-BENZOTRIAZOLYL TETRAMETHYLBUTYLPHENOL (Tinosorb® M de BASF) ; les triazines telles que celles identifiées par les noms INCI ETHYLHEXYL TRIAZONE (Uvinul® T150 de BASF) et BIS ETHYLHEXYLOXYPHENOL METHOXYPHENYL TRIAZINE (Tinosorb® S de BASF) ; les benzimidazoles, tels que celui identifié par le nom INCI PHENYLBENZIMIDAZOLE SULFONIC ACID (Eusolex® 232 de MERCK) ; les hydroxybenzoates, tels que l'ETHYLHEXYL SALICYLATE (Eusolex® OS de MERCK) et l'HOMOSALATE (Eusolex® HMS de MERCK) ; le BUTYL METHOXYDIBENZOYLMETHANE (Eusolex® 9020 de MERCK) ; l'ETHYLHEXYL METHOXYCINNAMATE (Uvinul® MC 80 de BASF) ; les cyanoacrylates, tels que l'OCTOCRYLENE (Eusolex® OCR de MERCK) ; et leurs mélanges.

Cette composition peut se présenter sous toute forme adaptée à une application topique sur la peau et notamment sous forme de lait, de crème, de lotion, de gel ou de masque. Il s'agit généralement d'une composition non rincée.

Cette composition peut constituer un produit de soin de la peau, notamment du visage, en particulier un produit anti-âge ou anti-rides ; un produit solaire ou après-solaire ; un produit autobronzant ; ou un produit de maquillage de la peau tel qu'un fond de teint. En variante, il peut s'agir d'une composition de nettoyage de la peau. On préfère que la composition utilisée selon l'invention constitue une composition anti-âge, anti-rides et/ou de protection solaire.

Cette composition peut être appliquée sur au moins une zone du corps d'une personne présentant des signes de vieillissement cutané (par exemple d'au moins 30 ans, voire d'au moins 40 ans), et plus particulièrement sur le visage, le cou et/ou le décolleté. En variante, elle peut être appliquée sur l'ensemble du corps. Elle peut par ailleurs être appliquée une ou plusieurs fois par jour, par exemple matin et/ou soir, sur les zones à traiter.

EXEMPLES

L'invention sera mieux comprise à la lumière des exemples suivants, qui sont donnés à titre purement illustratif et n'ont pas pour but de limiter la portée de l'invention, définie par les revendications annexées.

**Exemple 1** : **Effet anti-élastase d'une huile essentielle de rameaux de sapin blanc**

On a obtenu une huile essentielle de rameaux de sapin blanc de l'espèce *Abies alba* par hydrodistillation de rameaux (branches et aiguilles) récoltés dans la Drôme. L'hydrodistillation a été réalisée sous cohobation pendant 4h à partir de 1,15 kg de rameaux séchés et broyés.

L'huile essentielle obtenue a été analysée par chromatographie en phase gazeuse couplée à une spectrométrie de masse. Elle renfermait essentiellement du limonène (35%), de l'alpha-pinène (10,8%) et du camphène (12,3%) et, en plus faible quantité, de l'acétate de bornyle (4,6%).

Son activité d'inhibition de l'élastase a été évaluée *in vitro* par comparaison avec :

- un extrait commercial de *Rubus idaeus* L (Hydraberry de VITALAB), et
- un extrait alcoolique de rameaux de sapin blanc, obtenu par extraction à l'éthanol pendant 4h, sous agitation, de rameaux séchés et coupés finement.

Pour ce faire, l'enzyme, le substrat (Ala-Ala-Ala-p-nitroanilide) et l'échantillon sont mis en contact et la libération du peptide p-nitranilide induit par l'activité de l'élastase est suivie par spectrophotométrie, à une longueur d'onde de 410 nm.

Les tests ont été précisément réalisés comme suit :
150 $\mu$L d'une solution d'élastase pancréatique porcine, préparée à 0,171 U/mL dans un tampon Tris (de concentration 50 mM et de pH 8), ont été distribués dans des puits d'une plaque de 96 puits (Thermo Nunc) de façon à obtenir une concentration finale de l'enzyme par puits de 0,1 U/mL et on y a ajouté 7,5 $\mu$L d'extrait à évaluer (dilué à 3,433 mg/mL dans du DMSO) afin d'obtenir une concentration finale par puits de 100 $\mu$g/mL. Le DMSO seul constituait le contrôle négatif (DO$_{contrôle}$). La plaque a été scellée à l'aide de films adhésifs (Greiner Bio-One) et incubée durant 20 min à température ambiante. Une première lecture est réalisée à 410 nm afin d'obtenir la densité optique du blanc, à l'aide d'un lecteur de plaque Spectramax Plus 384 (Molecular Devices). Puis 100 $\mu$L de N-succinyl-Ala-Ala-Ala-p-nitroanilide (Suc-AAA-pNA) à 2,06 mM dans le tampon Tris ont été ajoutés dans chaque puits de la plaque (concentration finale en Suc-AAA-pNA de 0,8 mM par puits). Après 40 min d'incubation à température ambiante, l'absorbance a été mesurée à 410 nm afin d'évaluer le pourcentage d'inhibition. Pour ce faire, les données ont été acquises à l'aide du logiciel SoftMaxPro (Molecular Devices) et les pourcentages d'inhibition ont été calculés à l'aide du logiciel Prism (GraphPad Software), selon la formule suivante :

$$I\% = [(DO_{contrôle}-DO_{échantillon})/DO_{contrôle}] \times 100, \text{ DO renvoyant à la densité optique.}$$

Chaque valeur de densité optique a été corrigée avec la mesure du blanc, correspondant à l'absorbance de l'échantillon avant l'addition du substrat.

Tous les tests ont été menés en triplicats et les résultats sont présentés en pourcentage d'inhibition couplé à l'écart-type associé.

Résultats :

L'activité anti-élastase mesurée pour l'huile essentielle de sapin blanc est d'environ 100%, tandis qu'elle n'est que de 77,1 ± 0,095% pour le témoin positif et de 69,6 ± 0,015% pour l'extrait alcoolique.

**Exemple 2** : **Composition cosmétique**

La composition suivante est préparée de manière classique pour l'homme de l'art, en mélangeant les ingrédients ci-dessous dans les proportions pondérales indiquées.

| | |
|---|---|
| Huile essentielle selon l'Exemple 1 | 0,01-0,5 % |
| Adénosine | 0,05 % |
| Huiles | 5-15 % |
| Gélifiants huileux | 1-5 % |
| Gélifiants aqueux | 1-3 % |
| Polyols | 20-25 % |

(suite)

| | | |
|---|---|---|
| Séquestrant | | qs |
| Anti-oxydants | | qs |
| Parfum | | qs |
| Conservateurs | | qs |
| Eau | qsp | 100 % |

**Revendications**

1. Utilisation d'une huile essentielle de rameaux de sapin blanc *(Abies alba)* comme inhibiteur d'élastase pour lutter contre les signes du vieillissement cutané.

2. Utilisation selon la revendication 1, **caractérisée en ce que** l'huile essentielle est utilisée pour limiter et/ou réduire la dégradation des fibres élastiques de la peau, le relâchement cutané et/ou la perte d'élasticité de la peau et/ou l'apparence des rides.

3. Utilisation selon la revendication 1 ou 2, **caractérisée en ce que** l'huile essentielle est obtenue par hydrodistillation ou entraînement à la vapeur, de préférence par hydrodistillation, de rameaux de sapin blanc cultivé en Europe, de préférence en France.

4. Utilisation selon l'une quelconque des revendications 1 à 3, **caractérisée en ce que** l'huile essentielle renferme, comme constituant principal, du limonène, et, comme constituants secondaires, de l'alpha-pinène, du camphène et de l'acétate de bornyle, dans un rapport molaire du limonène à l'acétate de bornyle allant de 2:1 à 20:1, de préférence de 5:1 à 10:1, tel que mesuré par chromatographie en phase gazeuse.

5. Utilisation selon la revendication 4, **caractérisée en ce que** le rapport molaire du camphène à l'alpha-pinène est compris entre 0,5:1 et 2:1, de préférence entre 1:1 et 1,5:1 et/ou le rapport molaire du camphène à l'acétate de bornyle est compris entre 1:1 et 5:1, de préférence entre 2:1 et 3:1, tels que mesurés par chromatographie en phase gazeuse.

6. Utilisation selon l'une quelconque des revendications 1 à 5, **caractérisée en ce que** l'huile essentielle est incluse dans une composition cosmétique adaptée à une application topique sur la peau, de préférence une composition anti-âge, anti-rides et/ou de protection solaire.

7. Utilisation selon la revendication 6, **caractérisée en ce que** l'huile essentielle représente de 0,001 à 5% en poids et préférentiellement de 0,01 à 0,5% en poids, par rapport au poids total de la composition.

8. Procédé cosmétique pour lutter contre les signes du vieillissement cutané, notamment pour limiter et/ou réduire la dégradation des fibres élastiques de la peau, le relâchement cutané et/ou la perte d'élasticité de la peau et/ou l'apparence des rides, comprenant l'application topique sur la peau d'une composition cosmétique renfermant une huile essentielle de rameaux de sapin blanc *(Abies alba)*.

**Patentansprüche**

1. Verwendung eines ätherischen Öls von Zweigen der Weißtanne *(Abies alba)* als Elastaseinhibitor zur Bekämpfung der Symptome der Hautalterung.

2. Verwendung nach Anspruch 1, **dadurch gekennzeichnet, dass** das ätherische Öl zur Begrenzung und/oder Verminderung des Abbaus von elastischen Fasern der Haut, der Erschlaffung der Haut und/oder des Verlusts der Elastizität der Haut und/oder des Erscheinens von Falten verwendet wird.

3. Verwendung nach Anspruch 1 oder 2, **dadurch gekennzeichnet, dass** das ätherische Öl durch Wasserdampfdestillation oder Dampfdestillation, vorzugsweise durch Wasserdampfdestillation, von Zweigen der Weißtanne erhalten wird, die in Europa, vorzugsweise in Frankreich, angebaut wird.

**4.** Verwendung nach einem der Ansprüche 1 bis 3, **dadurch gekennzeichnet, dass** das ätherische Öl als Hauptbestandteil Limonen und als Nebenbestandteile alpha-Pinen, Camphen und Bornylacetat in einem Stoffmengenverhältnis von Limonen zu Bornylacetat von 2:1 bis 20:1, vorzugsweise von 5:1 bis 10:1, wie mittels Gaschromatographie gemessen, umfasst.

**5.** Verwendung nach Anspruch 4, **dadurch gekennzeichnet, dass** das Stoffmengenverhältnis von Camphen zu alpha-Pinen zwischen 0,5:1 und 2:1, vorzugsweise zwischen 1:1 und 1,5:1, beträgt und/oder das Stoffmengenverhältnis von Camphen zu Bornylacetat zwischen 1:1 und 5:1, vorzugsweise zwischen 2:1 und 3:1, wie mittels Gaschromatographie gemessen, beträgt.

**6.** Verwendung nach einem der Ansprüche 1 bis 5, **dadurch gekennzeichnet, dass** das ätherische Öl in eine kosmetische Zusammensetzung zum topischen Auftragen auf die Haut, vorzugsweise eine Anti-Aging-, Anti-Falten- und/oder Sonnenschutz-Zusammensetzung, eingeschlossen ist.

**7.** Verwendung nach Anspruch 6, **dadurch gekennzeichnet, dass** das ätherische Öl von 0,001 bis 5 Gew.-% und vorzugsweise von 0,01 bis 0,5 Gew.-%, bezogen auf das Gesamtgewicht der Zusammensetzung, darstellt.

**8.** Kosmetisches Verfahren zur Bekämpfung der Symptome der Hautalterung, insbesondere zur Begrenzung und/oder Verminderung des Abbaus von elastischen Fasern der Haut, der Erschlaffung der Haut und/oder des Verlusts der Elastizität der Haut und/oder des Erscheinens von Falten, das die topische Anwendung einer kosmetischen Zusammensetzung, die ein ätherisches Öl von Zweigen der Weißtanne *(Abies alba)* umfasst, auf die Haut umfasst.

**Claims**

**1.** The use of an essential oil of silver fir (*Abies alba*) branches as elastase inhibitor to combat the signs of skin aging.

**2.** The use as claimed in claim 1, **characterized in that** the essential oil is used to limit and/or reduce the degradation of the elastic fibers of the skin, skin slackening and/or the loss of skin elasticity and/or the appearance of wrinkles.

**3.** The use as claimed in claim 1 or 2, **characterized in that** the essential oil is obtained by hydrodistillation or steam distillation, preferably by hydrodistillation, of branches of silver fir grown in Europe, preferably in France.

**4.** The use as claimed in any one of claims 1 to 3, **characterized in that** the essential oil contains, as principal constituent, limonene, and, as secondary constituents, alpha-pinene, camphene and bornyl acetate, in a molar ratio of limonene to bornyl acetate ranging from 2:1 to 20:1, preferably from 5:1 to 10:1, as measured by gas chromatography.

**5.** The use as claimed in claim 4, **characterized in that** the molar ratio of camphene to alpha-pinene is comprised between 0.5:1 and 2:1, preferably between 1:1 and 1.5:1 and/or the molar ratio of camphene to bornyl acetate is comprised between 1:1 and 5:1, preferably between 2:1 and 3:1, as measured by gas chromatography.

**6.** The use as claimed in any one of claims 1 to 5, **characterized in that** the essential oil is included in a cosmetic composition suitable for topical application to the skin, preferably an anti-aging, anti-wrinkle and/or sun protection composition.

**7.** The use as claimed in claim 6, **characterized in that** the essential oil represents from 0.001 to 5% by weight and preferentially from 0.01 to 0.5% by weight, based on the total weight of the composition.

**8.** A cosmetic process for combating the signs of skin aging, in particular for limiting and/or reducing the degradation of the elastic fibers of the skin, skin slackening and/or the loss of skin elasticity and/or the appearance of wrinkles, comprising the topical application to the skin of a cosmetic composition containing an essential oil of silver fir (*Abies alba*) branches.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2017044050 A
- FR 1525945
- JP 2014224073 A

**Littérature non-brevet citée dans la description**

- **D.L.J. OPDYKE.** Monographs on Fragrance Raw Materials. Pergamon Press, 1979
- **SA YANG et al.** *Journal of Clinical Biochemistry and Nutrition,* 2009, vol. 44 (3), 253-259